# EUROPEAN PATENT SPECIFICATION

(11) **EP 2 606 853 B1**
(45) Date of publication and mention of the grant of the patent: **27.05.2020**
(21) Application number: 12198789.5
(22) Date of filing: 21.12.2012
(51) Int. Cl.: A61F 2/07

(54) **Hybrid aortic arch replacement**
Hybrider Aortenbogenersatz
Remplacement d'arc aortique hybride

(30) Priority: 22.12.2011 US 201113335103
(43) Date of publication of application: 26.06.2013
(73) Proprietor: Cook Medical Technologies LLC, Bloomington, IN 47404 (US)
(72) Inventor: Roeder, Blayne, Lafayette, Indiana 47909 (US)
(74) Representative: Powell, Stephen David

(56) References cited:
- EP-A1- 1 245 202
- EP-A1- 1 913 897
- WO-A1-2009/145913
- AU-B1- 2010 254 599
- CN-A- 102 166 143
- US-A1- 2006 004 433

## Description

### TECHNICAL FIELD

This disclosure relates to medical devices for implantation within the human or animal body for treatment of endovascular disease. More particularly, it relates to a system comprising a hybrid prosthesis adapted for treating a thoracic aorta of a patient, and an introducer.

### BACKGROUND

Endovascular methods have been proposed for treatment of aneurysms of the aorta, particularly when an aneurysm is adjacent the aorta bifurcation. But when an aneurysm occurs higher up in the aorta, for example, in the region of the descending aorta adjacent the thoracic arch or in the ascending aorta, endovascular techniques for treating these aneurysms are somewhat more difficult because of the arched nature of the thoracic arch, the existence of major arteries in the region, and the proximity to the heart.

Generally, operations to treat aneurysms that include the ascending aorta or the arch have been done by open chest surgery. Such surgery generally involves surgical replacement of a portion of the aorta with a tubular prosthesis. The surgery is a high risk procedure. Two foremost reasons for the risk associated with the procedure are difficulty of accessing the site of treatment and the potential for neural ischemia. The surgical Bentall technique has been demonstrated with some success for treating ascending aortic aneurysms. But the Bentall technique may be used only in patients able to tolerate a fully surgical technique and thus is not suitable for patients that may be intolerant of such an invasive procedure.

In dealing with aortic arch aneurysms, procedural risk is greatly increased by inclusion of the brachiocephalic vessels and the aorta distal to the arch. The difficulty of the procedure also may be exacerbated by the necessity to reconnect the left common carotid and left subclavian arteries to the tubular prosthesis after replacing a portion of the aorta. Although surgical techniques have been successfully demonstrated to repair arch aneurysms, such techniques are highly invasive and thus limited in utility, especially in high risk patients.

EP 1 913 897 A1 discloses a prosthesis comprising a distal graft portion including a tubular main body comprising a biocompatible material and a support

### SUMMARY

The present embodiments provide a hybrid prosthesis and systems for facilitating deployment of such a prosthesis.

According to a first aspect of the present invention, there is provided a system for the treatment of a vessel defect, the system comprising: a hybrid prosthesis comprising: a distal end graft portion configured to extend from the aortic arch into the descending aorta and including a tubular main body comprising a support structure, the distal graft portion having a proximal end, a distal end, and a main lumen extending therebetween, at least one tubular branch extending from the main body of the distal graft portion near the proximal end thereof and having a first end, a second end and a branch lumen extending therebetween, the branch lumen being in fluid communication with the main lumen, the tubular branch extending from the main body at an acute angle whereby the distal graft portion has a generally Y shaped configuration; and a proximal graft portion extending from the distal graft portion, wherein the main body of the distal graft portion and the body of the proximal graft portion are formed from a single piece of graft material, the proximal graft portion including a tubular body, the body of the proximal graft portion being corrugated and entirely unstented, having a proximal end, a distal end, and a lumen extending therebetween and being configured for placement within the ascending aorta and extending into the aortic arch; and an introducer comprising a central catheter and an auxiliary catheter, wherein at least a portion of the distal graft portion of the hybrid prosthesis is retained in a compressed configuration on the central catheter of the introducer for endoluminal delivery to a site of the vessel defect, and wherein the auxiliary catheter is positioned within the branch.

A method (not being part of the invention) employing the above system for treating a defect of a thoracic aorta of a patient may include making an incision in a thoracic arch of the patient. The incision may be positioned proximal to an ostium of a left subclavian artery of the patient. The method also may include introducing a distal graft portion of a hybrid prosthesis through the incision and into the thoracic aorta. The method also may include endoluminally positioning a distal end of the distal graft portion within a descending aorta of the patient and endoluminally positioning a branch of the distal graft portion within the left subclavian artery of the patient.

Other systems, features, and advantages of the invention will be, or will become, apparent to one with skill in the art upon examination of the following figures and detailed description. It is intended that all such additional systems, features, and advantages be within the scope of the invention, and be encompassed by the following claims.

### BRIEF DESCRIPTIONS OF THE DRAWINGS

FIG. 1 is a perspective view of one embodiment of a hybrid prosthesis.
FIG. 2 is a perspective view of an alternative embodiment of a hybrid prosthesis.
FIG. 3 is a longitudinal cross sectional view of an example of an introducer for a hybrid prosthesis useful in explaining the present invention.
FIG. 4 is a perspective view of one embodiment of an introducer for a hybrid prosthesis of an embodiment of a system in accordance with the present invention.
FIG. 5 depicts an initial configuration in the formation of a diameter reducing tie for a hybrid prosthesis.
FIG. 6 depicts the diameter reducing tie of FIG. 5 in its delivery configuration.
FIG. 7 is a schematic view of an aorta of a patient.
FIG. 8 illustrates one example of a method of delivering a distal graft portion of a hybrid prosthesis within an aorta of a patient.
FIG. 9 illustrates one example of a method of deploying a distal graft portion of a hybrid prosthesis within an aorta of a patient.
FIG. 10 illustrates one example of a method of placing a proximal graft portion of a hybrid prosthesis within an aorta of a patient.

### DETAILED DESCRIPTION OF THE DRAWINGS AND THE PRESENTLY PREFERRED EMBODIMENTS

The present disclosure relates to a hybrid prosthesis system.

In the present disclosure, the term "proximal" refers to a direction that is generally closest to the heart during a medical procedure, while the term "distal" refers to a direction that is farthest from the heart during a medical procedure.

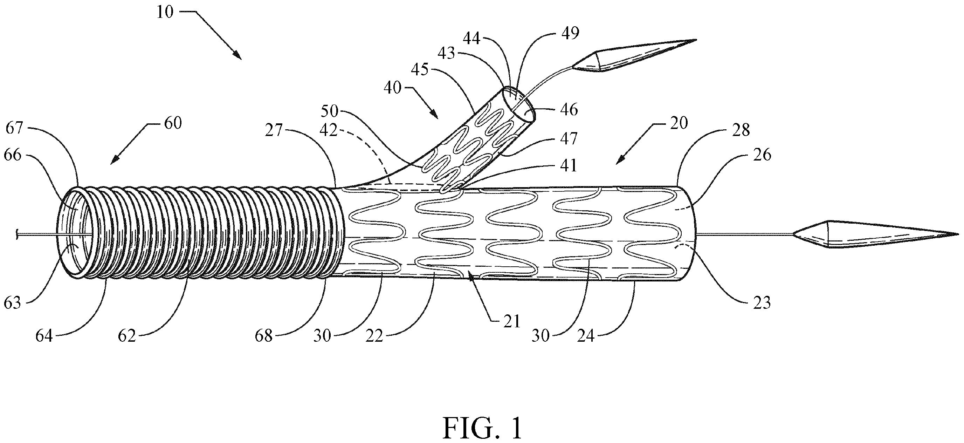

**FIG. 1** depicts one example of a hybrid prosthesis 10. The hybrid prosthesis 10 may include a distal graft portion 20 and a proximal graft portion 60. As further described below, the distal graft portion 20 may be generally configured as a stent graft, while the proximal graft portion 60 may be generally configured as a surgical graft.

The distal graft portion 20 may include a main body 21 and a branch 40. The main body 21 may include a substantially tubular graft body 22 having an inner surface 23 and an outer surface 24. The graft body 22 may be configured as a generally tubular member having a substantially cylindrical shape. The inner surface 23 of the graft body 22 may define a main lumen 26 extending longitudinally within the main body 21 between a proximal end 27 and a distal end 28 thereof. The main lumen 26 may be suitable for passing fluid such as, for example, blood therethrough.

The main body 21 of the distal graft portion 20 may include at least one support structure 30, such as a stent. The support structure 30 preferably is radially compressible for delivery of the distal graft portion 20 and radially expandable for deployment of the distal graft portion. The support structure 30 may be configured as a single, unitary structure or as a plurality of independent structures. The support structure 30 and/or various portions thereof may be disposed on the inner surface 23 and/or the outer surface 24 of the graft body 22. Multiple support structures 30 may be positioned at various locations along a length of the main body 21. A support structure 30 may be positioned near the distal end 28 of the distal graft portion 20 to seal the distal end against a wall of a body vessel. Additionally, or alternatively, a support structure 30 may be positioned near the proximal end 27 of the distal graft portion 20 and/or the point of attachment between the main body 21 and the branch 40. This support structure may aid in fixing the prosthesis 10 in place with respect to the body vessel and/or supporting the branch 40 extending from the main body 21. The support structures 30 positioned near the distal end 28 and the proximal end 27 of the main body 21 may be positioned on the inner surface of the graft body 22. The branch 40 of the distal graft portion 20 may have a first end 41 with a first end opening 42 and a second end 43 with a second end opening 44. The branch 40 may include a substantially tubular graft body 45 having an inner surface 46 and an outer surface 47. The graft body 45 may be configured as a generally tubular member having a substantially cylindrical shape. The inner surface 46 of the graft body 45 may define a branch lumen 49 extending longitudinally between the first end 41 and the second end 43 of the branch 40. The branch 40 may include at least one support structure 50. The support structure 50 may include a single, unitary structure or a plurality of independent structures. The support structure 50 and/or various portions thereof may be disposed on the inner surface 46 and/or the outer surface 47 of the graft body 45. Multiple support structures 50 may be positioned at various locations along a length of the branch 40.

The branch 40 may extend from the main body 21. To that end, the graft body 45 of the branch 40 may be formed integrally with the graft body 22 of the main body 21. Alternatively, the branch 40 may be formed separately, and the first end 41 of the branch may be attached to the graft body 22. The branch 40 extends from the main body 21 such that the distal graft portion 20 has a generally Y shaped configuration as shown in **FIG. 1****.** The branch 40 may be configured as a peripheral branch extending from a side of the main body 21 or a contralateral branch attached to a leg of a Y formed by the main body. The branch 40 extends from the main body 21 at an acute angle as shown in **FIG. 1****.** The branch 40 is attached to the main body 21 at a position that enables the branch to be aligned with a branching vessel as further described below. To that end, the branch 40 is attached to the main body 21 near the proximal end 27 of the main body.

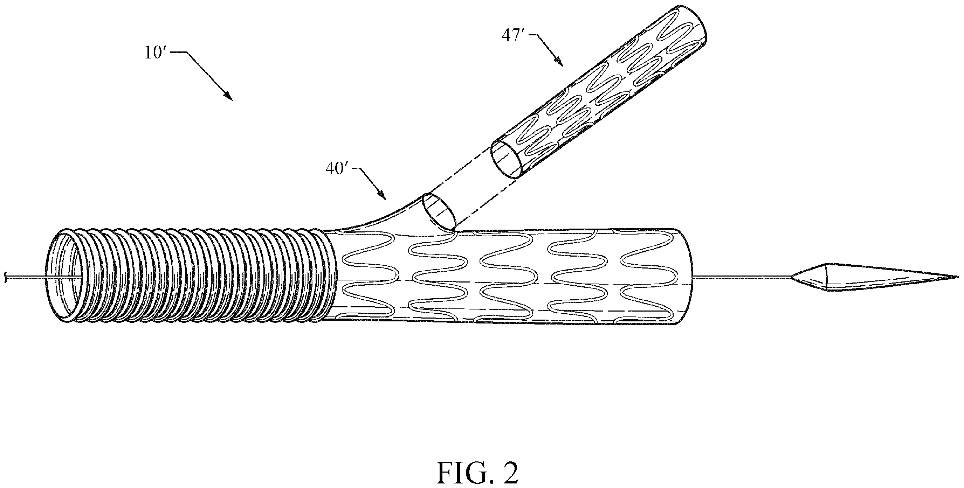

**FIG. 2** illustrates another embodiment of a prosthesis 10'. The prosthesis 10' may be substantially identical to the prosthesis 10 with the exception of the branch. The branch of the distal graft portion of the prosthesis 10' may be configured as an extension 40' as shown in **FIG. 2****.** The extension 40' may be relatively short as compared to the branch 40 of the prosthesis 10 and may be configured to receive a branch prosthesis 47. The branch prosthesis 47 may be configured as a stent graft. The branch prosthesis 47 may be deployed within the extension 40' using any suitable technique (e.g., endovascular techniques) and may be configured to extend, for example, into a left subclavian artery of a patient as described below with reference to the branch 40. Any discussion in this disclosure regarding the prosthesis 10 may be generally applicable to the prosthesis 10'.

Returning to **FIG. 1****,** the branch 40 may be attached to the main body 21 by sutures, wire, staples, clips, bonding agents, or other methods that may be used to achieve a secure attachment. For example, the branch 40 may be attached to the main body 21 by any method described in U.S. Patent Application Pub. No. 2006/0095118 by Hartley. The branch 40 may be attached to the graft body 22 and/or the support structure 30 of the main body 21. Preferably, the graft body 45 of the branch 40 may be attached to the graft body 22 of the main body 21 to form a fluid-tight seal. For example, the graft body 45 of the branch 40 may be stitched to the graft body 22 of the main body 21. An aperture may be formed in the graft body 22 of the main body 21. The aperture may be aligned with the first end opening 42 of the branch 40 to enable fluid communication between the main lumen 26 and the branch lumen 49 through the aperture. In this manner, the distal graft portion 20 of the prosthesis 10 may be configured to serve as a conduit for blood to flow through the main body and branch lumens 26, 49 from the proximal end 27 of the main body 21 to the distal end 28 of the main body 21 and the second end 43 of the branch 40.

The proximal graft portion 60 of the prosthesis 10 may include a substantially tubular graft body 62 having an inner surface 63 and an outer surface 64. The graft body 62 may be configured as a generally tubular member having a substantially cylindrical shape. The inner surface 63 of the graft body 62 may define a main lumen 66 extending longitudinally within the graft body between a proximal end 67 and a distal end 68 of the proximal graft portion 60. The main lumen 66 may be suitable for passing fluid such as, for example, blood therethrough. The proximal graft portion 60 does not include a support structure such as a stent. In other words, the proximal graft portion 60 is devoid of stents or unstented as shown in **FIG. 1****.**

The graft body 62 of the proximal graft portion 60 may be corrugated or crimped to increase flexibility and/or decrease the risk of kinking. Such corrugation or crimping also may help to promote setting of the proximal graft portion in a curved shape. Suitable crimps and methods for crimping the graft body 62 are described in U.S. Patent No. 7,407,509 and U.S. Patent Application Pub. No. 2005/0113905 to Greenberg et al.

The proximal graft portion 60 may extend proximally from the distal graft portion 20. To that end, the distal end 68 of the proximal graft portion 60 may be attached to the proximal end 27 of the distal graft portion 20. The graft body 62 of the proximal graft portion 60 and the graft body 22 of the distal graft portion 20 are formed integrally with one another as a unitary structure. In other words, the graft body 62 and the graft body 22 may be formed together from a single piece of graft material. The lumen 66 of the proximal graft portion 60 is in fluid communication with the main lumen 26 of the distal graft portion 20 to create a substantially continuous flow path between the proximal end 67 of the proximal graft portion and the distal end 28 of the distal graft portion. Although the proximal graft portion has been described with reference to **FIG. 1****,** the description is equally applicable to the proximal graft portion shown in **FIG. 2****.**

The prosthesis 10 may be sized and shaped for placement within the vasculature of a patient as further described below. The preferred size and shape of the prosthesis 10 depends on the anatomy in which it is to be implanted. Physiological variables, deployment characteristics, and other factors also may contribute to the determination of a proper size and shape of the prosthesis 10. For example, the prosthesis 10 may have a size and shape suitable for placement in the ascending aorta, aortic arch, and/or descending aorta. To that end, the proximal graft portion 60 is configured for placement within the ascending aorta and extending into the aortic arch. The distal graft portion 20 may be configured for placement within the aortic arch and extending into the descending aorta. The branch 40 is configured to extend from the aortic arch into the left subclavian artery. The main body 21 of the distal graft portion 20 may have a diameter, for example, ranging from about 10 mm to about 46 mm. The diameter of the main body 21 may be constant along the length thereof. Alternatively, the main body 21 may be tapered such that the diameter of the main body may vary along the length thereof. The branch 40 may have a diameter, for example, ranging from about 8 mm to about 14 mm. The diameter of the branch 40 may be constant along the length thereof. Alternatively, the branch 40 may be tapered such that the diameter of the branch may vary along the length thereof. The main body 61 of the proximal graft portion 60 may have a diameter, for example, ranging from about 20 mm to about 40 mm. The diameter of the main body 61 may be constant along the length thereof. Alternatively, the main body 61 may be tapered such that the diameter of the main body may vary along the length thereof. In one example, main body 21 of the distal graft portion 20 may have a diameter that is different (e.g., larger or smaller) than the diameter of the main body 61 of the proximal graft portion 60. In this example, the distal end of the main body 61 and/or the proximal end of the main body 21 may be tapered so that the distal graft portion 20 and the proximal graft portion 60 may be attached or formed integrally with one another as described above. The prosthesis 10 may be deployed in combination with various other prostheses to effectively bridge an aneurysmal portion of the vasculature.

It is further contemplated that a distal graft portion of a prosthesis may have multiple branches extending from a main body. For example, the prosthesis may have two, three, or more branches extending from the main body. The various branches may be attached to the main body at different longitudinal and/or circumferential positions with respect to the main body. In this manner, the branches may be configured to extend into the left subclavian, left common carotid, and/or innominate arteries. Additionally, or alternatively, the prosthesis may be configured for placement at various other positions within the vasculature of the patient.

The graft bodies 22, 45, 62 may be made of any material known in the art. The graft bodies may be made of the same or different materials. Preferably, the graft bodies may be formed from a biocompatible material that is substantially non-toxic in the in vivo environment of its intended use and substantially unrejected by the patient's physiological system (i.e., is non-antigenic). For example, the graft bodies may be made of an expanded polytetrafluoroethylene (ePTFE), polytetrafluoroethylene, silicone, polyurethane, polyamide (nylon), polyethylene, polypropylene, polyaramids, polyacrylonitrile, and cellulose, as well as other flexible biocompatible materials. The graft bodies also can be made of known fabric graft materials such as woven polyester such as DACRON® from Invista (Wichita, Kansas), polyetherurethanes such as THORALON® from Thoratec Corporation (Pleasanton, California), or polyethylene such as an ultra-high molecular weight polyethylene (UHMwPE) such as DYNEEMA® from DSM Dyneema LLC (Stanley, North Carolina). In addition, materials that are not inherently biocompatible may be subjected to surface modifications to render the materials biocompatible. Examples of surface modifications include, for example, graft polymerization of biocompatible polymers on the surface, coating of the surface with a crosslinked biocompatible polymer, chemical modification with biocompatible functional groups, and immobilization of a compatibilizing agent such as heparin or other biocompatible substances. Thus, any fibrous material having sufficient strength to survive in the in vivo environment may be used to form a textile graft, provided the final textile is biocompatible. Additionally, or alternatively, the graft bodies may include a bioremodelable material such as reconstituted or naturally-derived collagenous materials, extracellular matrix (ECM) material, submucosa, renal capsule membrane, dermal collagen, dura mater, pericardium, fascia lata, serosa, peritoneum or basement membrane layers, or intestinal submucosa, including small intestinal submucosa (SIS), stomach submucosa, urinary bladder submucosa, and uterine submucosa. One non-limiting example of a suitable remodelable material is SURGISIS® BIODESIGN™ from Cook Medical (Bloomington, Indiana). Another suitable remodelable material is the graft prosthesis material described in U.S. Patent No. 6,206,931 to Cook et al. Additionally, or alternatively, the graft bodies may be made of any of the materials described in U.S. Patent No. 7,407,509 to Greenberg et al. or U.S. Patent Application Pub. No. 2009/0171451 by Kuppurathanam et al.

The support structures 30, 50 and/or various portions thereof may be compressible and/or expandable stents having any stent pattern known in the art. The stents may be balloon expandable. Preferably, the stents may be self-expandable. A self-expandable stent may have a relaxed state corresponding to an expanded configuration. The stents can maintain the patency of the prosthesis and ensure adequate sealing against the surrounding vascular tissue. Some of the goals for stent design and placement, whether internal or external, may include preventing metal-to-metal contact points, preventing contact between two different types of alloys, and minimizing micromotion. Preferably, stent sizing, spacing, and design may be determined so that there is no stent-to-stent contact even in tortuous anatomy. Stents preferably may be placed to maximize prosthesis flexibility while maintaining patency, as well as reducing material wear and stent fatigue. Furthermore, it is preferable that the stents do not interfere with the branch, that they minimize the potential for galvanic corrosion, and ensure adequate joint stability. Stent amplitude, spacing, and stagger preferably may be optimized for each prosthesis design. Any of the stents mentioned herein may have barbs and/or other anchoring members to help decrease prosthesis migration.

One example of a stent pattern is the Z-stent or Gianturco stent design. Each Z-stent may include a series of substantially straight segments, or struts, interconnected by a series of bent segments, or bends. The bent segments may include acute bends or apices. The Z-stents may be arranged in a zigzag configuration in which the straight segments are set at angles relative to one another and are connected by the bent segments. This design provides both significant radial force as well as some longitudinal support. In tortuous anatomy or branches, it may be preferable to use alternative stents or modifications to the Z-stent design to avoid stent-to-stent contact. Alternative stents may include, for example, annular or helical stents. Furthermore, in complex anatomical situations, external stents have the potential to become intertwined with the wires and other devices utilized to ensure branch vessel access, sealing, and fixation. In some instances, it may be desirable to affix some of the stents to the internal surface of the prosthesis.

The stents described herein may be made from any suitable material known in the art. In one example, the stents may be made from standard medical grade stainless steel and may be soldered using silver standard solder (0 lead/0 tin). In other examples, the stents may be made from a metallic material selected from stainless steel, silver, platinum, palladium, gold, titanium, tantalum, iridium, tungsten, cobalt, chromium, cobalt-chromium alloy 1058, cobalt-based 35N alloy, nickel-based alloy 625, a molybdenum alloy, a molybdenum alloy including about 0.4% to about 0.8% of lanthanum oxide (La₂O₃), and a nickel-titanium alloy, such as nitinol, or other suitable materials known in the art. Additionally, or alternatively, the stents may be made from nitinol or other shape-memory metal. Moreover, the stents may be configured in a variety of ways to provide a suitable intraluminal support structure. For example, one or more stents may be made from a woven wire structure, a laser-cut cannula, individual interconnected rings, or another pattern or design.

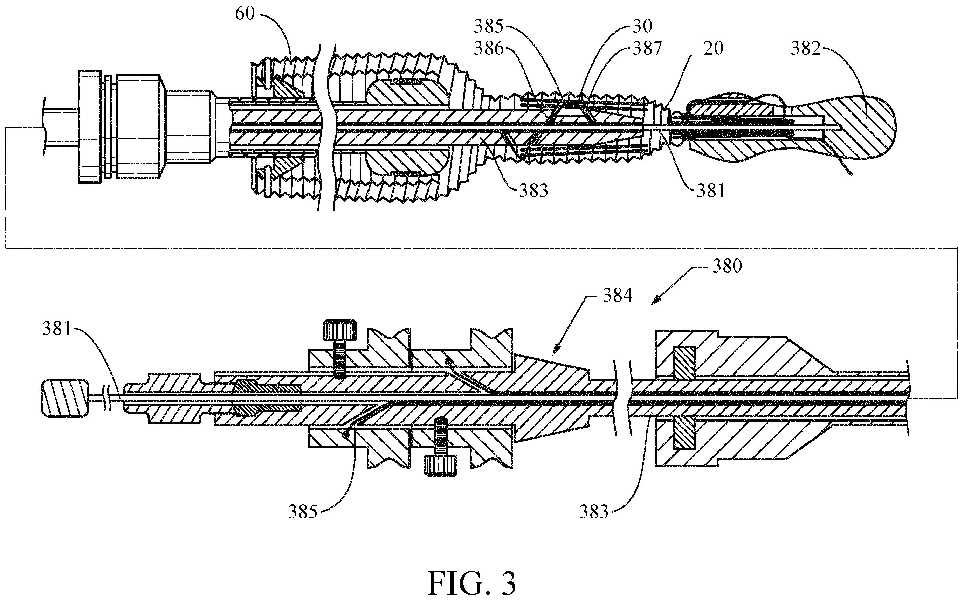

Referring now to **FIGS. 3-9****,** exemplary systems and method steps for using the prosthesis of **FIG. 1** to treat a condition in the area of a patient's ascending aorta, aortic arch, and/or descending aorta are shown and described. Although **FIGS. 3-9** will be described with reference to the prosthesis shown in **FIG. 1****,** the description is generally applicable to the prosthesis shown in **FIG. 2****.**

The prosthesis 10 may be compressed into a delivery configuration and mounted onto a deployment device such as an introducer. Any type of deployment device suitable for deploying a branched stent graft may be used. For example, suitable deployment devices may include those described in U.S. Patent Nos. 7,488,344 and 7,537,606 to Hartley et al.; and U.S. Patent Application Pub. No. 2004/0193244 by Hartley et al.*.* The operator may directly manipulate the introducer, which may provide the operator with a relatively high degree of control during the procedure. Further, such deployment devices may be compact and may have a relatively uniform, low-diameter radial profile, allowing for atraumatic access and delivery.

**FIG. 3** illustrates one example of an introducer 380 useful in explaining the present invention. The introducer 380 may include a central catheter 381 extending from a proximal end of the introducer to a tip 382 at a distal end of the introducer. In use, the introducer may be introduced into the aorta from an incision in the patient's chest and advanced distally as further described below. In such a procedure, the proximal portion of the introducer may correspond generally to the proximal end of the prosthesis and the distal portion of the introducer may correspond generally to the distal end of the prosthesis. A deployment catheter 383 may surround the central catheter 381. The deployment catheter 383 may be moved longitudinally with respect to the central catheter 381 and may be locked into position with respect to the central catheter in known fashion by means of a pin vice arrangement at the proximal end of a handle 384. The handle 384 may be positioned at the proximal end of the deployment catheter 383 and may remain outside of the patient during use of the introducer.

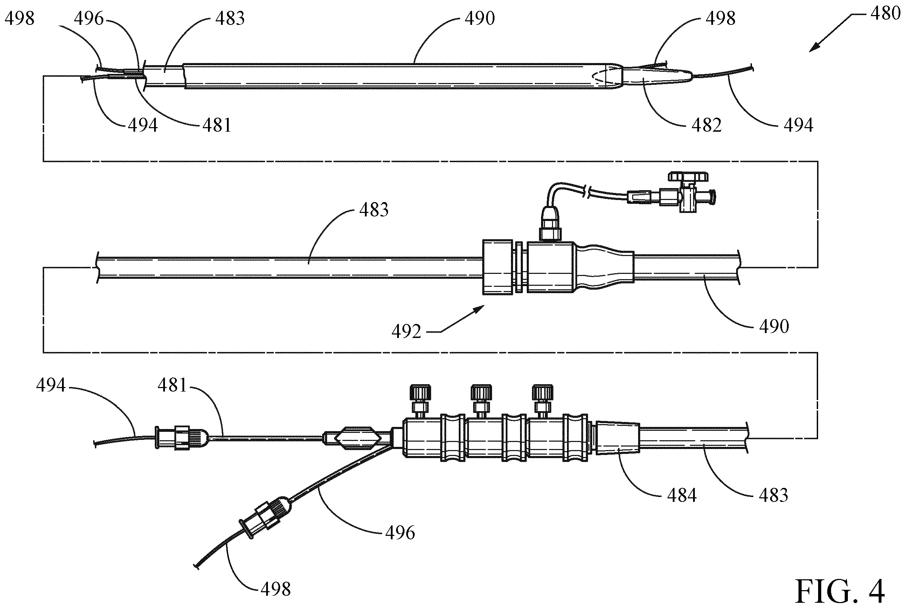

**FIG. 4** illustrates an introducer 480 of a system in accordance with the present invention. The introducer 480 may be configured substantially as described in U.S Patent Application Pub. No. 2009/0254170 by Hartley et al. For example, the introducer 480 may include a central catheter 481 received within a lumen of a deployment catheter 483. A sheath 490 may extend distally from a sheath manipulator 492 to cover at least a portion of the deployment catheter 483. A nose cone 482 may be attached to the distal end of the central catheter 481, and a handle 484 may be positioned near the proximal end of the central catheter. A guide wire 494 may extend within the central catheter 481. The introducer 480 may include an auxiliary catheter 496 with an auxiliary guide wire 498 received therein. The auxiliary guide wire 498 may be positioned within the branch 40 of the prosthesis 10 to cannulate a branch vessel in any known manner. A set of trigger wire release arrangements may be mounted on the handle 484 to manipulate a series of trigger wires as further described below.

To load the prosthesis 10 in an introducer (e.g., the introducer 380 or the introducer 480), the distal end of the introducer may be inserted distally into the lumen 66 of the proximal graft portion 60 and advanced distally within the prosthesis 10 until the distal end of the introducer exits the distal end 28 of the distal graft portion 20. The sheath is be positioned around the distal graft portion 20 of the prosthesis 10 to retain the distal graft portion in a compressed, delivery configuration.

When the prosthesis 10 is loaded on an introducer, the proximal graft portion 60 of the prosthesis 10 may be positioned around a portion of the central catheter proximal of the distal graft portion 20. The proximal graft portion 60 may be gathered, folded, or otherwise arranged in a reduced diameter and/or a reduced length configuration around the central catheter. In some examples, the proximal graft portion 60 of the prosthesis 10 may be positioned within the sheath of an introducer.

As shown in **FIG. 3****,** the prosthesis 10 may be releasably attached to the introducer 380 to temporarily fix the prosthesis in place relative to the introducer until positioned as desired within the patient's body. For example, one or more trigger wires may engage a portion of the prosthesis 10 and may be releasably attached to the introducer 380 as further described below.

When mounted on the delivery device, at least the distal graft portion 20 of the prosthesis 10 may be compressed into a reduced diameter delivery configuration. In other words, the support structure 30 of the main body 21 may be compressed at least in a radial direction. Additionally, or alternatively, the support structure 50 of the branch 40 may be compressed at least in a radial direction. The support structures 30 and 50, respectively, may be radially expanded by a suitable device applying a radially outward force or may be self-expanding once a retaining means is removed after positioning the graft in the intended location. In one example, the prosthesis 10 may be radially compressed over the central catheter 381 of the introducer 380. The main body 21 and/or the branch 40 of the distal graft portion 20 may be retained in the delivery configuration by any suitable means. Suitable means for retaining the distal graft portion 20 in the delivery configuration may include those described in U.S. Patent No. 5,456,713 to Chuter and U.S. Patent Application Pub. Nos. 2006/0004433 by Greenberg et al.; 2007/0142896 by Anderson et al.; 2008/0114438 by Hartley et al.; and 2008/0294234 by Hartley et al.

In one example, a trigger wire 385 may extend along a length of the delivery catheter. The trigger wire 385 may extend between the central catheter 381 and the deployment catheter 383 within the lumen of the deployment catheter from the handle 384 to the distal end of the introducer 380. The trigger wire 385 may extend through a side aperture 386 in the deployment catheter 383 to exit the deployment catheter to engage and retain a stent 30 of the distal graft portion 20 in a compressed configuration. The trigger wire 385 may directly engage the stent 30 to retain the stent in close proximity to the introducer 380 (i.e., in the compressed configuration). Additionally, or alternatively, the trigger wire 385 may engage one or more reducing members such as diameter reducing ties or mooring loops to retain the stent 30 in the compressed configuration. After the trigger wire 385 engages the stent 30, the trigger wire may re-enter another side aperture 387 in the deployment catheter and extend further distally out of the distal end 28 of the distal graft portion 20 of the prosthesis 10. The trigger wire 385 may be received within one or more apertures of the tip 382 to retain the distal end of the trigger wire and to prevent the trigger wire from fouling with other objects during deployment of the prosthesis 10. The trigger wire 385 may be held in tension between the handle 384 and the tip 382 to oppose the outward radial force of the support structure 30 to retain at least a portion of the distal graft portion 20 in the compressed configuration. Releasing the trigger wire 385 may enable expansion of the distal graft portion 20 as further described below.

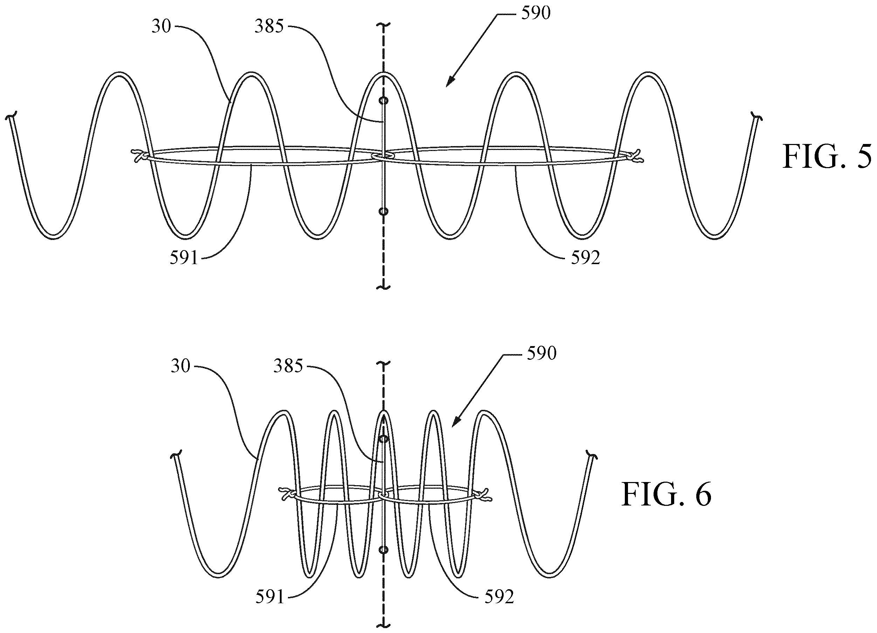

**FIGS. 5-6** illustrate the application of one example of a diameter reducing tie 590 that may be applied to the prosthesis 10. A first thread 591 may be passed around the trigger wire 385 so that opposing end portions of the first thread are extended out to one side of the trigger wire. Each of the end portions of the first thread 591 may pass over a series of intermediate struts of the stent 30. One of the end portions then may pass over a target strut and the other end portion may pass under the target strut, as shown in **FIG. 5****,** so that the first thread 591 forms a loop around the trigger wire 385 and the target strut. The two end portions of the first thread 591 may be tied in a knot, which may be pulled tight as shown in **FIG. 6** so that the intermediate struts between the trigger wire 385 and the knot are pulled closer together. Excess portions of the first thread 591 extending beyond the knot may be trimmed as shown in **FIG. 6****.** A second thread 592 may be applied in a similar manner. The thread 592 may be passed around the trigger wire 385 or around the two strands of the first thread 591 and across the trigger wire. Opposing end portions of the second thread 592 may be extended out to the other side of the trigger wire, opposite the first thread 591, over a series of intermediate struts, and looped around a target strut as shown in **FIG. 5****.** The two end portions of the second thread 592 may be tied in a knot, which may be pulled tight as shown in **FIG. 6** so that the intermediate struts between the trigger wire 385 and the knot are pulled closer together. In this manner, the circumference of the stent 30 may be restrained so that the diameter of at least a portion of the distal graft portion 20 of the prosthesis 10 may remain reduced for delivery within the patient.

The trigger wire 385 may be stitched longitudinally along at least a portion of the prosthesis 10 (e.g., the main body 21 of the distal graft portion 20) to engage the diameter reducing ties or mooring loops applied to the prosthesis. Stitching the trigger wire into the graft material of the prosthesis may render the deployment catheter unnecessary. In other words, one or more trigger wires may be threaded through the graft material of the prosthesis so that the prosthesis itself guides the trigger wires. In this manner, positioning the trigger wires between the central catheter and the deployment catheter may not be required, and the deployment catheter may be omitted from the introducer.

Although the trigger wire 385 has been described as retaining only a single stent of the distal graft portion 20 of the prosthesis 10 in the compressed configuration, the trigger wire may engage any or all of the stents along a length of the main body 21 of the distal graft portion in similar fashion. For example, multiple pairs of apertures may be provided in the deployment catheter 383 so that the trigger wire 385 may exit and re-enter the deployment catheter at multiple points along a length thereof. The apertures may be aligned with the stents 30 so that the trigger wire 385 may engage a stent (or a diameter reducing tie or mooring loop) adjacent each pair of apertures. Additionally, or alternatively, multiple trigger wires may be provided. Each trigger wire may engage one or more stents, diameter reducing ties, or mooring loops. In this manner, the main body 21 of the distal graft portion 20 of the prosthesis 10 may be retained in the compressed configuration along substantially an entire length thereof.

Additional trigger wires, diameter reducing ties, and/or mooring loops may be provided along a length of the branch 40 of the distal graft portion 20 of the prosthesis 10. Separate trigger wires may be provided for the main body 21 and/or the branch 40. In this manner, the main body 21 and the branch 40 may be selectively expanded as desired by the operator. To deploy the prosthesis 10, or the distal graft portion 20 thereof, the operator may pull or retract one or more trigger wires, thereby releasing the trigger wires from the stents, diameter reducing ties, and/or mooring loops to enable outward expansion of at least a portion of the distal graft portion 20 of the prosthesis 10. Although use of trigger wires, diameter reducing ties, and mooring loops has been described in reference to the example of the introducer shown in **FIG. 3****,** the description is generally applicable to the introducer of the system in accordance with the present invention shown in **FIG. 4****.** Any other suitable delivery device may be used to deploy the prosthesis 10. For example, the prosthesis 10 or a portion of the prosthesis (e.g., the distal graft portion 20) may be received within a conventional peel-away sheath. The prosthesis 10 within the peel-away sheath may be loaded onto a positioner and introduced within a body vessel using any suitable method. With the distal graft portion 20 of the prosthesis 10 in a desired position within the body vessel, the peel-away sheath may be removed to enable expansion of the distal graft portion of the prosthesis.

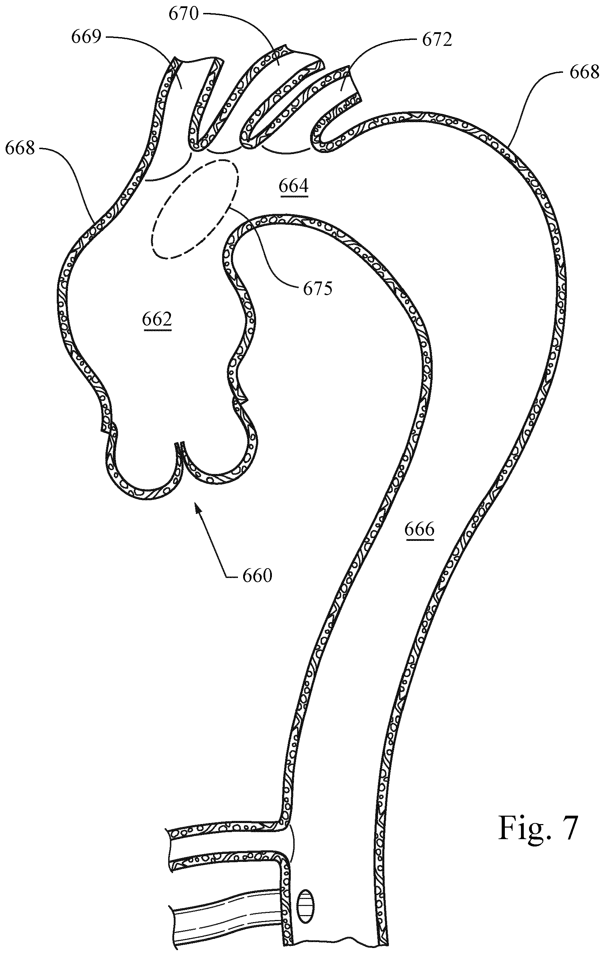

**FIG. 7** shows a schematic view of a thoracic arch region of an aorta of a patient. The aorta extends from an aortic valve 660 of the patient via the ascending aorta 662 to the thoracic arch 664 before proceeding down the descending aorta 666. An aneurysm 668 has been depicted in the ascending aorta as well as adjacent the thoracic arch 664 in the descending aorta 666. In the arch region 664, major arteries including the innominate artery 669, the left common carotid artery 670, and the subclavian artery 672 branch off from the aorta. Any deployment of a prosthesis into the aorta preferably may maintain blood perfusion to these arteries.

After cooling the patient's body temperature, exposing the aortic arch through a sternotomy, and performing cardiopulmonary bypass or systemic circulatory arrest, an incision 675 may be made in the side of the thoracic arch 664. As shown in **FIG. 7****,** the incision may extend from a portion of the thoracic arch 664 adjacent the ostia of the innominate artery 669 and left common carotid artery 670 to the distal portion of the ascending aorta 662. However, because the distal graft portion 20 of the prosthesis 10 may be deployed using endovascular techniques, as further described below, it may be unnecessary to expose the ostium of the left subclavian artery 672 or the proximal portion of the descending aorta 666.

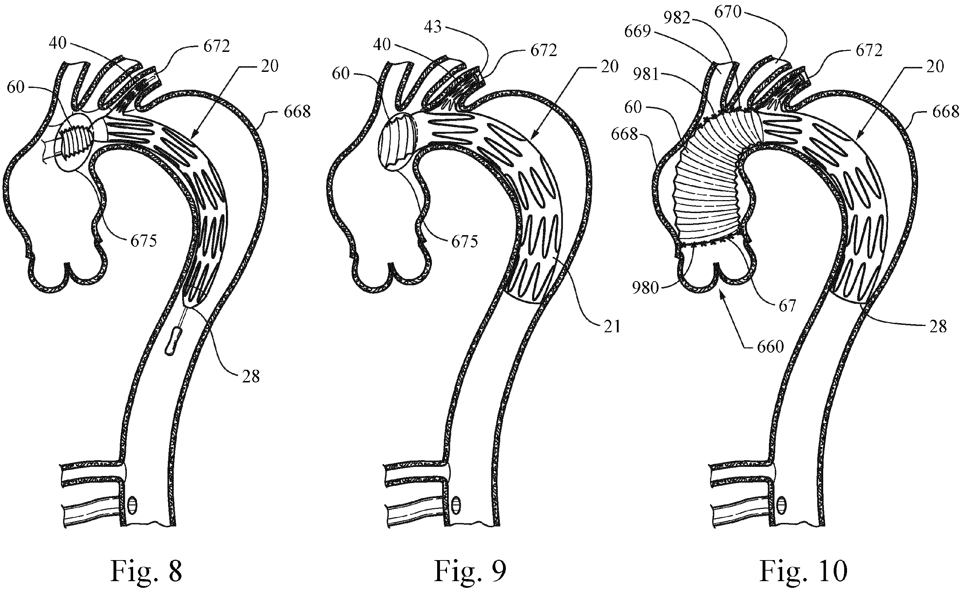

The distal graft portion 20 of the prosthesis 10 may be deployed using endovascular techniques. The prosthesis 10 may be placed transapically as shown in FIGS. **8-10**. For example the distal graft portion 20 of the prosthesis 10, in the delivery configuration and mounted on the distal end of the introducer 380, may be inserted through the incision 675, through the aortic arch 664, and into the descending aorta 666. A proximal portion of the introducer (e.g., the handle of the introducer) may remain external to the aorta throughout deployment of the prosthesis 10. With the distal graft portion 20 of the prosthesis disposed within the aorta, a delivery device such as an introducer, guide wire, catheter, or dilator may be inserted into the prosthesis 10 to cannulate the left subclavian artery. For example, the delivery device may be inserted into the prosthesis 10 from the proximal end 67 of the proximal graft portion 60. The delivery device may be advanced distally through the lumen 66 of the proximal graft portion 60 and into the lumen 26 of the main body 21 of the distal graft portion 20. The delivery device may be further advanced through the lumen 49 of the branch 40 to exit the second end 43 of the branch. A tip of the delivery device may be inserted through the ostium of the left subclavian artery 672 to cannulate the left subclavian artery. The introducer 380 and the prosthesis 10 may be advanced distally within the descending aorta 666 until the distal end 28 of the distal graft portion 20 is positioned distal to the aneurysmal region 668 as shown in **FIG. 8****.** In this position, the branch 40 of the distal graft portion 20 may be generally aligned in the vicinity of the ostium of the left subclavian artery 672. The proximal graft portion 60 of the prosthesis 10 may extend through the incision 675 to remain substantially external of the aorta. The proximal graft portion 60 may remain accessible for direct manipulation by the physician.

The distal graft portion 20 of the prosthesis 10 may be expanded as shown in **FIG. 9****.** The main body 21 may be expanded by, for example, retracting a trigger wire to disengage the stents of the main body or to release the diameter reducing ties or mooring loops retaining the main body in the compressed configuration. Additionally, or alternativley, the main body 21 may be expanded by retracting a sheath of the introducer in a known manner. Upon expansion of the main body 21, the branch 40 of the distal graft portion 20 may move distally along the delivery device positioned within the left subclavian artery 672 such that the second end 43 of the branch 40 may be moved to a position within the left subclavian artery. The branch 40 may be expanded by, for example, retracting a trigger wire to disengage the stents of the branch or to release the diameter reducing ties or mooring loops retaining the branch in the compressed configuration. The deployment device and/or delivery device may be retracted and removed from the patient's body following deployment of the distal graft portion 20 of the prosthesis 10.

Following deployment of the distal graft portion 20, the proximal graft portion 60 of the prosthesis 10 may be placed into the aorta using surgical techniques. For example, the proximal graft portion 60 may be manually introduced into the incision 675 in the thoracic arch 664 and directed into the ascending aorta 662 toward the aortic valve 660 as shown in **FIG. 10****.** The proximal end 67 of the proximal graft portion 60 may be sutured circumferentially at a suture 980 around the aortic valve 660 so that blood may flow out of the valve and into the prosthesis 10. An incision may be made through the graft material of the proximal graft portion 60 adjacent the ostium of the innominate artery 669. The proximal graft portion 60 may be sutured circumferentially at a suture 981 around the ostium of the innominate artery 669 so that blood may flow out of the prosthesis 10 and into the innominate artery. Another incision may be made through the proximal graft portion 60 adjacent the ostium of the left common carotid artery 670. The proximal graft portion 60 may be sutured circumferentially at a suture 982 around the ostium of the left common carotid artery 670 so that blood may flow out of the prosthesis 10 and into the left common carotid artery. In this manner, a substantially continuous flow pathway may be provided from the proximal end 67 of the proximal graft portion 60 of the prosthesis 10 into the innominate artery 669, the left common carotid artery 670, and the left subclavian artery 672, as well as to the distal end 28 of the distal graft portion 20 of the prosthesis 10. Any type of surgical fastening may be used instead of or in addition to the sutures described above. The proximal graft portion may be surgically fastened to the aorta by, for example, sutures, wire, staples, clips, bonding agents, or other methods that may be used to achieve a secure attachment. The aneurysmal portions 668 of the aorta may be substantially excluded by the prosthesis 10. The incision 675 and the chest cavity may then be closed.

Both endovascular and surgical techniques may be employed to perform the hybrid procedure described above. Such a hybrid procedure may have several advantages. For example, the use of endovascular techniques to deploy the distal graft portion of the prosthesis may make direct access to the ostium of the left subclavian artery or the proximal portion of the descending aorta by the physician unnecessary. Such direct access may be unnecessary because the procedure may not require sutures to be made around the ostium of the left subclavian artery or the proximal portion of the descending aorta. Direct access also may be unnecessary because the branch of the distal graft portion of the prosthesis may be inserted and deployed within the left subclavian artery from the proximal end of the proximal graft portion of the prosthesis. In other words, the physician may guide the branch into place from outside of the aorta using endovascular techniques and without directly accessing the left subclavian artery. The procedure, therefore, may be performed through a relatively small incision near the distal portion of the ascending aorta and/or the proximal end of the thoracic arch. The ostium of the left subclavian artery and the proximal portion of the descending aorta may remain substantially directly inaccessible throughout the procedure. In other words, because of the size and position of the incision, the ostium of the left subclavian artery and the proximal portion of the descending aorta may remain unexposed throughout the procedure. This may result in a less invasive procedure that may be performed on patients who may be intolerant of more invasive procedures such as Bentall or total arch replacement procedures.

The hybrid procedure described above also may be performed in a relatively short amount of time as compared to other procedures such as Bentall or total arch replacement procedures. For example, a physician may have difficulty reaching the ostium of the left subclavian artery to suture a graft thereto, as may be required by a procedure such as a total arch replacement procedure. A physician also may have difficulty reaching the proximal portion of the descending aorta. Such difficulty may be a result of the anatomy of the patient. For example, the position of the aorta may transition from an anterior position within the patient's body at the proximal portion of the thoracic arch to a posterior position at the proximal portion of the descending aorta. In other words, the proximal portion of the descending aorta may be positioned deeper within the patient's chest than the proximal portion of the thoracic arch. This may make direct access to the distal portion of the thoracic arch (e.g., the left subclavian artery) and/or the proximal portion of the descending aorta difficult for the physician. Because the distal graft portion of the prosthesis may be positioned without directly accessing the distal portion of the thoracic arch or the proximal portion of the descending aorta, direct access to the portions of the aorta that may be more difficult to reach may be unnecessary. Thus, the hybrid procedure may be performed in a relatively short amount of time as compared to other surgical procedures.

While various embodiments of the invention have been described, the invention is not to be restricted except in light of the attached claims.

Moreover, the advantages described herein are not necessarily the only advantages of the invention and it is not necessarily expected that every embodiment of the invention will achieve all of the advantages described.

## Claims

1. A system for the treatment of a vessel defect, the system comprising:
a hybrid prosthesis (10) comprising:
a distal end graft portion (20) configured to extend from the aortic arch into the descending aorta and including a tubular main body (21) comprising a support structure (30), the distal graft portion having a proximal end (27), a distal end (28), and a main lumen (26) extending therebetween, at least one tubular branch (40) extending from the main body of the distal graft portion near the proximal end (27) thereof and having a first end (41), a second end (42), and a branch lumen (49) extending therebetween, the branch lumen being in fluid communication with the main lumen, the tubular branch (40) extending from the main body (21) at an acute angle whereby the distal graft portion (20) has a generally Y shaped configuration; and
a proximal graft portion (60) extending from the distal graft portion,
wherein the main body (21) of the distal graft portion and the body (62) of the proximal graft portion are formed from a single piece of graft material, the proximal graft portion including a tubular body (62), the body of the proximal graft portion being crimped and entirely unstented, having a proximal end (67), a distal end (68), and a lumen (66) extending therebetween and being configured for placement within the ascending aorta and extending into the aortic arch; and
an introducer (480) comprising a central catheter (481) and an auxiliary catheter (496),
wherein at least a portion of the distal graft portion of the hybrid prosthesis is retained in a compressed configuration on the central catheter (481) of the introducer for endoluminal delivery to a site of the vessel defect, and
wherein the auxiliary catheter (496) is positioned within the branch (40).

2. The system of claim 1, further comprising at least one reducing member (590) attached to the support structure of the distal graft portion to retain at least a portion of the distal graft portion in the compressed configuration.

3. The system of claim 2, further comprising at least one trigger wire (385) threaded through a graft material of the distal graft portion and engaging the reducing member.

4. The system of any of claims 1 to 3, wherein the tubular branch (40) further comprises a support structure (50).

5. The system of claim 4, further comprising at least one reducing member attached to the support structure of the branch to retain at least a portion of the branch in the compressed configuration.

6. A system according to any of claims 1 to 5, wherein the support structure (30) of the distal graft portion comprises a plurality of stents.

## Patentansprüche

1. System für die Behandlung eines Gefäßdefekts, wobei das System Folgendes umfasst:
eine Hybrid-Prothese (10), umfassend:
einen distalen Graft-Abschnitt (20), der dazu ausgestaltet ist, sich von dem Aortenbogen in die absteigende Aorta zu erstrecken, und einen röhrenförmigen Hauptkörper (21) aufweist, der eine Stützstruktur (30) umfasst, wobei der distale Graft-Abschnitt ein proximales Ende (27), ein distales Ende (28) und ein sich dazwischen erstreckendes Hauptlumen (26) hat,
wobei sich mindestens ein röhrenförmiger Zweig (40) von dem Hauptkörper des distalen Graft-Abschnitts in der Nähe dessen proximalen Endes (27) erstreckt und ein erstes Ende (41), ein zweites Ende (42) und ein sich dazwischen erstreckendes Zweiglumen (49) hat, wobei das Zweiglumen in Fluidverbindung mit dem Hauptlumen steht, wobei sich der röhrenförmige Zweig (40) in einem spitzen Winkel von dem Hauptkörper (21) erstreckt, wodurch der distale Graft-Abschnitt (20) eine allgemein Y-förmige Konfiguration hat, und
einen proximalen Graft-Abschnitt (60), der sich von dem distalen Graft-Abschnitt erstreckt, wobei der Hauptkörper (21) des distalen Graft-Abschnitts und der Körper (62) des proximalen Graft-Abschnitts aus einem einzigen Stück Graft-Material gebildet sind, wobei der proximale Graft-Abschnitt einen röhrenförmigen Körper (62) aufweist, wobei der Körper des proximalen Graft-Abschnitts gecrimpt und
vollkommen stentlos ist und ein proximales Ende (67), ein distales Ende (68) und ein sich dazwischen erstreckendes Lumen (66) hat und zur Platzierung in der aufsteigenden Aorta ausgestaltet ist und sich in den Aortenbogen
erstreckt, und
eine Einführvorrichtung (480), die einen mittleren Katheter (481) und einen Hilfskatheter (496) umfasst,
wobei mindestens ein Abschnitt des distalen Graft-Abschnitts der Hybrid-Prothese für die endoluminale Zuführung zu einem Ort des Gefäßdefekts in einer komprimierten Konfiguration an dem mittleren Katheter (481) der Einführvorrichtung gehalten wird und wobei der Hilfskatheter (496) in dem Zweig (40) positioniert ist.

2. System nach Anspruch 1, ferner umfassend mindestens ein Reduzierglied (590), das an der Stützstruktur des distalen Graft-Abschnitts angebracht ist, um mindestens einen Abschnitt des distalen Graft-Abschnitts in der komprimierten Konfiguration zu halten.

3. System nach Anspruch 2, ferner umfassend mindestens einen Auslöserdraht (385), der durch ein Graft-Material des distalen Graft-Abschnitts gefädelt ist und das Reduzierglied in Eingriff nimmt.

4. System nach einem der Ansprüche 1 bis 3, wobei der röhrenförmige Zweig (40) ferner eine Stützstruktur (50) umfasst.

5. System nach Anspruch 4, ferner umfassend mindestens ein Reduzierglied, das an der Stützstruktur des Zweigs angebracht ist, um mindestens einen Abschnitt des Zweigs in der komprimierten Konfiguration zu halten.

6. System nach einem der Ansprüche 1 bis 5, wobei die Stützstruktur (30) des distalen Graft-Abschnitts eine Vielzahl von Stents umfasst.

## Revendications

1. Système pour le traitement d'un défaut de vaisseau, le système comprenant :
une prothèse hybride (10) comprenant :
une partie greffon d'extrémité distale (20) conçue pour s'étendre depuis l'arc aortique dans l'aorte descendante et comprenant un corps tubulaire principal (21) comprenant une structure de support (30), la partie greffon distale possédant une extrémité proximale (27), une extrémité distale (28) et une lumière principale (26) s'étendant entre celles-ci, au moins une ramification tubulaire (40) s'étendant depuis le corps principal de la partie greffon distale près de l'extrémité proximale (27) de celle-ci et possédant une première extrémité (41), une seconde extrémité (42) et une lumière de ramification (49) s'étendant entre celles-ci, la lumière de ramification étant en communication fluidique avec la lumière principale, la ramification tubulaire (40) s'étendant depuis le corps principal (21) selon un angle aigu moyennant quoi la partie greffon distale (20) possède une configuration généralement en forme de Y ; et
une partie greffon proximale (60) s'étendant depuis la partie greffon distale, dans lequel le corps principal (21) de la partie greffon distale et le corps (62) de la partie greffon proximale sont formés à partir d'un seul morceau de matériau de greffon, la partie greffon proximale comprenant un corps tubulaire (62), le corps de la partie greffon proximale étant serti et entièrement sans stent, possédant une extrémité proximale (67), une extrémité distale (68) et une lumière (66) s'étendant entre celles-ci et étant conçu pour un placement à l'intérieur de l'aorte ascendante et s'étendant dans l'arc aortique ; et
un introducteur (480) comprenant un cathéter central (481) et un cathéter auxiliaire (496),
dans lequel au moins une partie de la partie greffon distale de la prothèse hybride est retenue dans une configuration comprimée sur le cathéter central (481) de l'introducteur pour une mise en place endoluminale sur un site du défaut de vaisseau, et dans lequel le cathéter auxiliaire (496) est positionné à l'intérieur de la ramification (40).

2. Système selon la revendication 1, comprenant en outre au moins un élément de réduction (590) fixé à la structure de support de la partie greffon distale pour retenir au moins une partie de la partie greffon distale dans la configuration comprimée.

3. Système selon la revendication 2, comprenant en outre au moins un fil déclencheur (385) vissé à travers un matériau de greffon de la partie greffon distale et entrant en prise avec l'élément de réduction.

4. Système selon l'une quelconque des revendications 1 à 3, dans lequel la ramification tubulaire (40) comprend en outre une structure de support (50).

5. Système selon la revendication 4, comprenant en outre au moins un élément de réduction fixé à la structure de support de la ramification pour retenir au moins une partie de la ramification dans la configuration comprimée.

6. Système selon l'une quelconque des revendications 1 à 5, dans lequel la structure de support (30) de la partie greffon distale comprend une pluralité de stents.
